(19) Europäisches Patentamt
European Patent Office
Office européen des brevets

(11) Publication number: 0 342 341 A2

(12) # EUROPEAN PATENT APPLICATION

(21) Application number: 89105596.4

(22) Date of filing: 30.03.89

(51) Int. Cl.4: C07H 15/203 , A61K 31/70 , C12P 19/62 , //(C12P19/62, C12R1:29)

(30) Priority: 17.05.88 US 194783

(43) Date of publication of application: 23.11.89 Bulletin 89/47

(84) Designated Contracting States: AT BE CH DE ES FR GB GR IT LI NL SE

(71) Applicant: AMERICAN CYANAMID COMPANY
1937 West Main Street P.O. Box 60
Stamford Connecticut 06904-0060(US)

(72) Inventor: McGahren, William James
64 Glenwood Avenue
Demarest New Jersey 07627(US)
Inventor: Ellestad, George A.
59 Lt. Cox Drive
Pearl River New York 10965(US)

(74) Representative: Wächtershäuser, Günter, Dr.
Tal 29
D-8000 München 2(DE)

(54) **Process for producing antitumor antibiotic LL-E33288Gamma2.**

(57) This disclosure describes a novel aerobic fermentation process for producing the antitumor antibiotics LL-E33288$\gamma_2$-Br and LL-E33288$\gamma_2$-I.

EP 0 342 341 A2

# PROCESS FOR PRODUCING ANTITUMOR ANTIBIOTIC

## BACKGROUND OF THE INVENTION

The family of antibacterial and antitumor agents known collectively as the LL-E33288 complex are described and claimed in a series of related U.S. patent applications, namely, Serial No. 672,031, filed November 16, 1984; Serial No. 787,066, filed October 17, 1985; and Serial No. 009,321, filed January 30, 1987.

These applications describe the LL-33288 complex, the components thereof, namely, LL-E33288$\alpha_1$-Br, LL-E33288$\alpha_1$-I, LL-E33288$\alpha_2$-Br, LL-E33288$\alpha_2$-I, LL-E33288$\alpha_3$-Br, LL-E33288$\alpha_3$-I, LL-E33288$\alpha_4$-Br, LL-E33288$\beta_1$-Br, LL-E33288$\beta_1$-I, LL-E33288$\beta_2$-Br, LL-E33288$\beta_2$-I, LL-E33288$\gamma_1$-Br, LL-E33288$\gamma_1$-I, and LL-E33288$\delta_1$-I, and methods for their production by aerobic fermentation utilizing a new strain of Micromonospora echinospora ssp calichensis or natural or derived mutants thereof.

## SUMMARY OF THE INVENTION

This invention is concerned with a process of producing antitumor antibiotic LL-E33288$\gamma_2$ either as the iodo or bromo analog by aerobic fermentation.

LL-E33288$\gamma_2$-I active as an antitumor agent as shown in the following tests.

### Lymphocytic Leukemia P388 Test

The animals used were BDF$_1$ female mice. There were 5 or 6 mice per test group. The tumor transplant was by intraperitoneal injection of 0.5 ml of dilute ascitic fluid containing 1 x $10^6$ cells of lymphocytic leukemia P388 on day zero. The test compound was administered intraperitoneally at a volume of 0.5 ml in sterile, pyrogen free saline on days 1, 5 and 9 (relative to tumor inoculation) at the indicated doses. The mice were weighed and survivors recorded on a regular basis for 30 days. The median survival time for treated (T)/control (C) animals was calculated. The results appear in Table I.

Table I

| Lymphocytic Leukemia P388 Test | | | |
|---|---|---|---|
| Compound | Dose (mg/kg) | Median Survival (Days) | T/Cx100 % |
| LL-E33288$\gamma_2$-I | 0.02 | 19.5 | 177 |
| | 0.0125 | 21 | 191 |
| | 0.01 | 19 | 173 |
| | 0.0063 | 26 | 236 |
| | 0.005 | 21 | 191 |
| | 0.003 | 20.5 | 178 |
| | 0.0025 | 18.5 | 168 |
| | 0.0015 | 17 | 154 |
| | 0.00125 | 15.5 | 141 |
| Control | - | 11 | - |

### Melanotic Melanoma B16 Test

The animals used were BDF1 female mice. There were 5 or 6 mice per test group. A one gram portion

of melanotic melanoma B16 tumor was homogenized in 10 ml of cold balanced salt solution and a 0.5 ml aliquot of the homogenate was implanted intraperitoneally into each of the test mice. The test compound was administered intraperitoneally on days 1 through 9 (relative to tumor inoculation) at the indicated doses. The mice were weighed and survivors recorded on a regular basis for 60 days. The median survival time and the ratio of survival time for treated (T)/control (C) animals were calculated. The results appear in Table II.

TABLE II

| Melanotic Melanoma B16 Test | | | |
|---|---|---|---|
| Compound | Dose (mg/kg) | Median Survival (Days) | T/C x 100 (%) |
| LL-E33288$\gamma_2$-I | 0.005 | 34 | 162 |
| | 0.0025 | 38 | 181 |
| | 0.00125 | 33.5 | 160 |
| | 0.0006 | 28.5 | 136 |
| | 0.0003 | 26 | 124 |
| Control | - | 21 | - |

LL-E33288$\gamma_2$-I has an ultraviolet spectrum as shown in Figure I, a proton magnetic resonance spectrum as shown in Figure II, an infrared spectrum as shown in Figure III, and a mass spectrum as shown in Figure IV.

LL-E33288$\gamma_2$-I has a proposed structure as shown below.

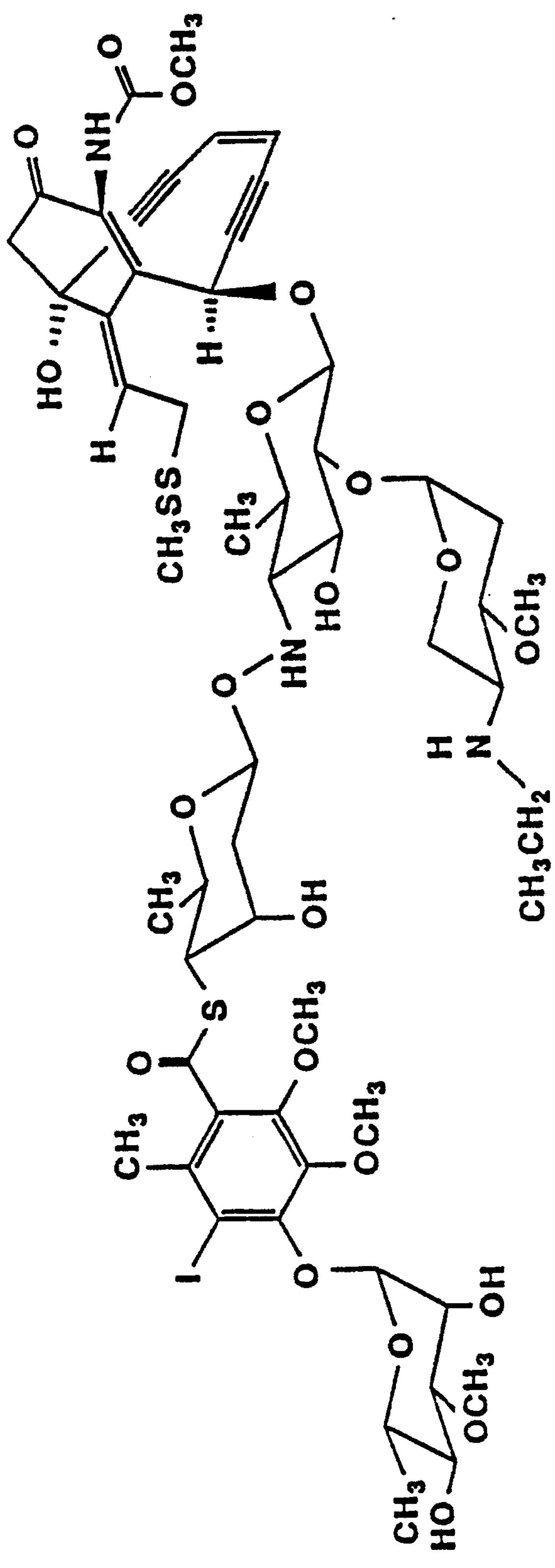

LL-E33288 $_{2}$-I

## DESCRIPTION OF THE INVENTION

The antitumor agents LL-E33288$\gamma_2$-I and LL-E33288$\gamma_2$-Br may be formed during the cultivation under controlled conditions of a new strain of Micromonospora echinospora ssp. calichensis. This microorganism

is maintained in the culture collection of the Medical Research Division, American Cyanamid Company, Pearl River, New York, as culture number LL-E33288. A viable culture of this new microorganism has been deposited with the Culture Collection Laboratory, Northern Regional Research Center, U.S. Department of Agriculture, Peoria, Ill. and has now bee added to its permanent collection under the strain designation NRRL-15839. Two derived mutants, LL-E33288-R66 (NRRL-15975) and LL-E33288-UV784 (NRRL-18149), which also produce LL-E33288$\gamma_2$-I and LL-E33288$\gamma_2$-Br have also been deposited with this repository. Access to said cultures under the above strain designations during pendency of the instant application shall be available to one determined by the Commissioner of Patents and Trademarks to be entitled thereto under C.F.R. § 1.14 and 35 U.S.C. § 122, and all restrictions on availability to the public of such cultures will be irrevocably removed upon grant of a U.S. patent on the instant application.

## General Fermentation Conditions

Cultivation of *Micromonospora echinospora* ssp. *calichensis* NRRL-15839, 15975, or 18149 may be carried out in a wide variety of liquid culture media. Media which are useful for the production of LL-E33288$\gamma_2$-I and LL-E33288$\gamma_2$-Br include an assimilable source of carbon such as starch, sugar, molasses, glycerol, etc.; an assimilable source of nitrogen such as protein, protein hydrolysate, polypeptides, amino acids, corn steep liquor, etc.; and inorganic anions and cations, such as potassium, sodium, ammonium, calcium, sulfate, carbonate, phosphate, chloride, etc.; and sources of bromine such as sodium bromide (if the bromo derivative is desired) or iodine such as potassium iodide (if the iodo derivative is desired). Trace elements such as boron, molybdenum, copper, etc., are supplied as impurities of other constituents of the media. Aeration in tanks and bottles is supplied by forcing sterile air through or onto the surface of the fermenting medium. Further agitation in tanks is provided by a mechanical impeller. An antifoam agent such as a silicone may be added as needed.

The invention will be described in greater detail in conjunction with the following non-limiting specific examples.

## Example 1

### Inoculum Preparation

A typical medium used to grow the primary inoculum was prepared according to the following formula:

| | |
|---|---|
| Beef extract | about 0.3% |
| Tryptone | about 0.5% |
| Dextrose | about 0.5% |
| Dextrin | about 2.4% |
| Calcium carbonate | about 0.4% |
| Yeast extract | about 0.5% |
| Water | qs to 100% |

This medium was adjusted to pH 7.0 and then sterilized. A 100 ml portion of this sterile medium, in a flask, was inoculated with frozen mycelia of the culture NRRL-15839. The inoculated medium was placed on a rotary shaker and agitated vigorously for 48 hours at 32°C. This incubated medium was then used to inoculate 10 liters of the above sterile medium in a 14 liter fermentor. This medium was incubated, with agitation, at 32°C for 48 hours, providing secondary inoculum. This secondary inoculum was then used to inoculate 300 liters of above sterile medium in a tank and incubated for 48 hours at 30° C while agitated by an impeller driven at 180-220 rpm, providing the tertiary or seed inoculum.

## Example 2

## Tank Fermentation

A fermentation medium was prepared according to the following formulation:

| | |
|---|---|
| Dextrose | about 0.5% |
| Sucrose | about 1.5% |
| Peptone, bacteriological grade | about 0.2% |
| Dibasic potassium phosphate | about 0.01% |
| Molasses | about 0.5% |
| Calcium carbonate | about 0.5% |
| Source of iodine* | trace amounts |
| Water | qs to 100% |

*If the LL-E33288$\gamma_2$-Br were to be produced, trace amounts of a bromine source should be used.

A 2800 liter portion of the above medium was sterilized and then inoculated with 300 liters of tertiary (seed) inoculum prepared as described in Example 1. Aeration was supplied at the rate of 0.53 liters of sterile air per liter of mash per minute and agitation was supplied by an impeller driven at 110 rpm. The temperature was maintained at about 28°C and the fermentation was terminated after about 97 hours, at which time the mash was harvested.

The fermentation was monitored for production of the LL-E33288$\gamma_2$-I by antibacterial activity, biochemical induction assay, TLC and HPLC analyses.

## Example 3

### Preliminary Isolation of a Mixture of LL-E33288$\gamma_2$-I and LL-E33288$\delta_1$-I

The whole harvest mash from a fermentation conducted as described in Example 2 was adjusted to pH 6 and then extracted with 1/2 the mash volume of ethyl acetate. The ethyl acetate extract was desolventized and then defatted by partitioning between methanol and hexane. The methanolic phase was concentrated to dryness. The resultant viscous gum was taken up in methylene chloride and subjected to clean-up chromatography over silica gel. The desired metabolites were eluted off using a stepwise gradient of from 3 to 5% methanol in methylene chloride. The recovered solids containing the $\gamma_1$-I, $\gamma_2$-I, $\delta_1$-I and $\epsilon$-I metabolites were resolved using a Waters LC/500 preparative instrument. The chromatography was carried out using a reverse phase Prepak cartridge with a solvent system consisting of 45 parts acetonitrile and 55 parts of 0.2M ammonium acetate solution (pH 7.0). A material recovered following this chromatography was a mixture of LL-E33288$\gamma_2$-I and LL-E33288$\delta_1$-I.

## Example 4

### Isolation of LL-E33288$\gamma_2$-I

A 250 mg portion of the product of Example 3, containing LL-E33288$\gamma_2$-I and LL-E33288$\delta_1$-I, was charged on a 1 inch packed column of $C_{18}$ Bondapak support (25$\mu$ particle size) and subjected to HPLC using a Separations Technology Lab 800A instrument. The solvent system consisted of 35 parts of acetonitrile and 65 parts of 0.1M ammonium acetate (pH 4.5) buffer. The pH of the ammonium acetate was adjusted using acetic acid. Fractions of 200 ml each were collected. The chromatography was monitored at

245 mm. Fractions 2 and 3 were combined, desolventized and extracted with ethyl acetate. This extract was dried, then concentrated to a few ml and added dropwise with stirring to 20 ml of hexane. The precipitate was collected and dried, giving 13 mg of LL-E33288$\gamma_2$-I.

## Example 5

### Isolation of LL-E33288$\gamma_2$-I

A 104 mg portion of the product of Example 3, containing LL-E33288$\gamma_2$-I and LL-E33288$\delta_1$-I, was dissolved in 3 ml of acetonitrile. To this solution was added a solution of 11.2 mg of dithiothreitol in 0.2 ml of acetonitrile. After 2.5 hours, this mixture was filtered, the filtrate diluted to 7.5 ml and charged on 1 inch packed column of $C_{18}$ Bondapak as described in Example 4. The column was developed with acetonitrile: 0.1M ammonium acetate (pH 4.5) buffer (35:65), monitored at 245 mm and 200 ml fractions were taken. Fractions 3-6 were combined, desolventized and extracted with ethyl acetate. The extract was dried, concentrated to 2-3 ml and added dropwise to 20 ml of hexane. The resultant precipitate was collected and dried at high vacuum at the temperature of boiling acetone giving 13 mg of LL-E33288$\gamma_2$-I as an off-white solid.

## Example 6

### Isolation of LL-E33288$\gamma_2$-I

A 250 mg portion of the product of Example 3, containing LL-E33288$\gamma_2$-I and LL-E33288$\delta_1$-I, was dissolved in 5.5 ml of acetonitrile. To this solution was added a solution of 25 mg of dithiothreitol in 0.5 ml of acetonitrile. The reaction was allowed to stand for 1.5 hours and then filtered. The filtrate was concentrated to 3-4 ml and an equal volume of 0.1M ammonium acetate (pH 4.5) buffer was added. This mixture was chromatographed as described in Example 5, developing with acetonitrile: 0.1M ammonium acetate (pH 4.5) buffer (38:62). The elution was monitored at 245 mm and 200 ml fractions taken. Fractions 3, 4 and 5 were combined, dried, desolventized and extracted with ethyl acetate. The extract was dried, concentrated to a few ml and added dropwise with stirring to 20 ml of hexane. The precipitate was collected and dried, giving 25 mg of LL-E33288$\gamma_2$-I as an off-white solid.

## Claims

1. A process for producing antitumor antibiotic LL-E33288$\gamma_2$-I which comprises aerobically fermenting the organism Micromonospora echinospora ssp calichensis NNRL-15839, or mutants thereof, including NRRL-15975 and NRRL-18149 in a liquid medium containing assimilable sources of carbon, nitrogen, iodine, and inorganic salts, until substantial antibiotic activity is imparted to said medium and then recovering LL-E33288$\gamma_2$-I therefrom.

2. A process for producing antitumor antibiotic LL-E33288$\gamma_2$-I which comprises aerobically fermenting a liquid medium containing assimilable sources of carbon, nitrogen, iodine and inorganic salts; which medium has been inoculated with a viable culture of the organism Micromonospora echinospora ssp calichensis NRRL-15839 or mutants thereof including NRRL-15975 and NRRL-18149, maintaining said fermentation culture at a temperature of about 24-32°C for a period of approximately 90-200 hours, harvesting the mash and extracting the LL-E33288$\gamma_2$-I therefrom.

3. A process for producing antitumor antibiotic LL-E33288$\gamma_2$-Br which comprises aerobically fermenting the organism Micromonospora echinospora ssp calichensis NRRL-15839, or mutants thereof, including NRRL-15975 and NRRL-18149 in a liquid medium containing assimilable sources of carbon, nitrogen, bromine, and inorganic salts, until substantial antibiotic activity is imparted to said medium and then recovering LL-E33288$\gamma_2$-Br therefrom.

4. A process for producing antitumor antibiotic LL-E33288$\gamma_2$-Br which comprises aerobically fermenting a liquid medium containing assimilable sources of carbon, nitrogen, bromine and inorganic salts; which medium has been inoculated with a viable culture of the organism Micromonospora, echinospora ssp calichensis NRRL-15839 or mutants thereof including NRRL-15975 and NRRL-18149, maintaining said fermentation culture at a temperature of about 24-32°C for a period of approximately 90-200 hours, harvesting the mash and extracting the LL-E33288$\gamma_2$-I therefrom.

5. A process for extracting LL-E33288$\gamma_2$ from a whole harvest mash derived as described in any of claims 1-4, which comprises the steps of:

a) adjusting the mash to pH 6 and extracting with 1/2 of the mash volume of ethyl acetate;

b) desolventizing and defatting the ethyl acetate extract by partitioning between methanol and hexane;

c) desolventizing the methanol portion, dissolving in methylene chloride and chromatographing on silica gel, eluting with a stepwise gradient of from 3 to 5% methanol in methylene chloride, giving a mixture of LL-E33288$\gamma_1$-, LL-E33288$\gamma_2$, LL-E33288$\delta_1$ and LL-E33288$\epsilon$;

d) resolving these components by reverse phase chromatography with the solvent system acetonitrile: 0.2M ammonium acetate (pH 7.0) (45:55) giving a mixture of LL-E33288$\gamma_2$ and LL-E33288$\delta_1$; and

e) resolving this mixture by chromatography using acetonitrile: 0.1M ammonium acetate (pH 4.5), giving LL-E33288$\gamma_2$.

6. A process for separating LL-E33288$\gamma_2$ from a mixture of LL-E33288$\gamma_2$ and LL-E33288$\delta_1$ which comprises the steps of:

a) dissolving the mixture in acetonitrile;

b) reacting with a solution of dithiothreitol in acetonitrile;

c) separation by chromatography, eluting with acetonitrile: 0.1M ammonium acetate (pH 4.5) (35:65);

d) desolventizing the active fractions and extracting with ethyl acetate;

e) adding the extract to hexane and collecting the LL-E33288$\gamma_2$.

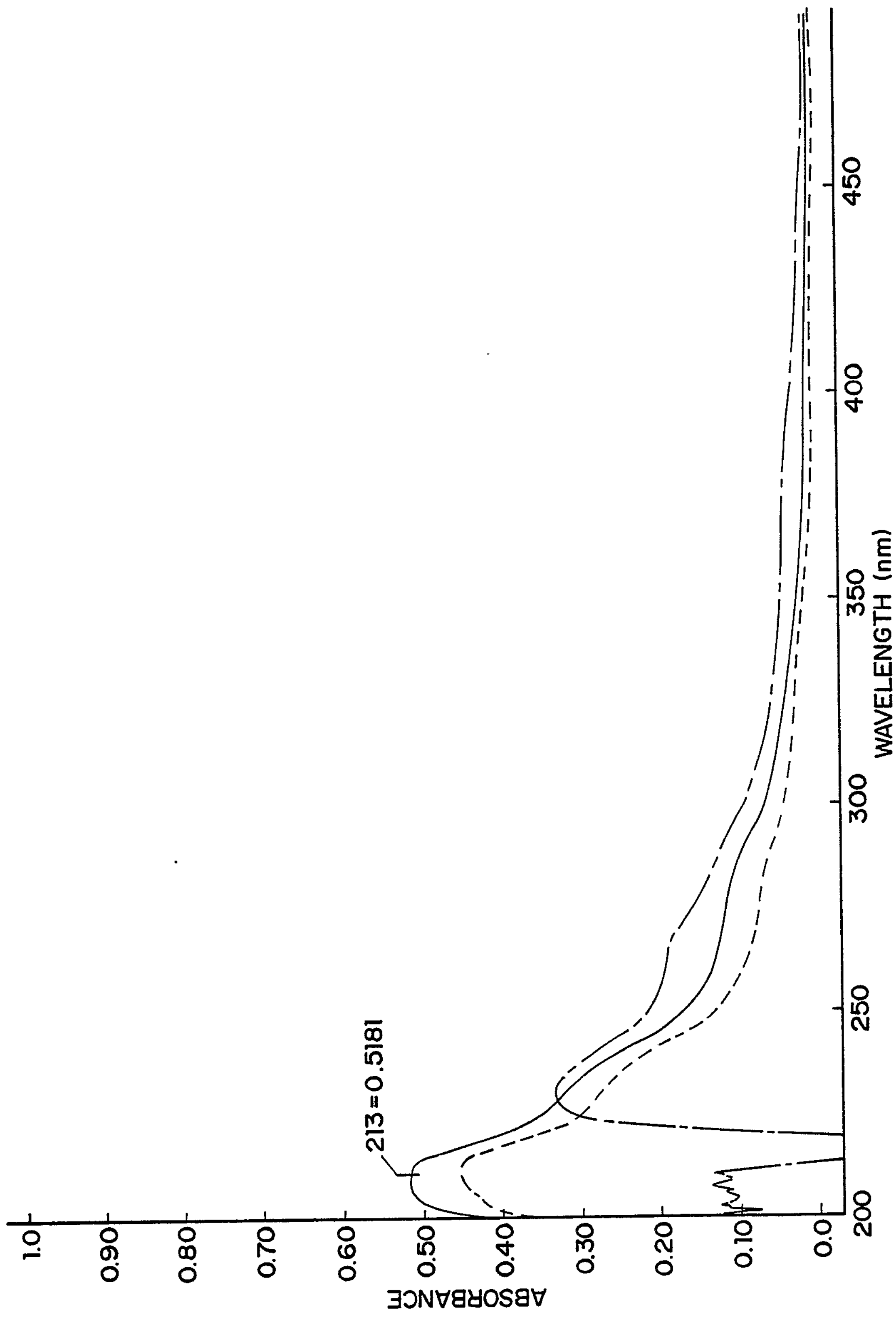
ABSORBANCE
1.0
0.90
0.80
0.70
0.60
0.50
0.40
0.30
0.20
0.10
0.0
213=0.5181
200
250
300
350
400
450
WAVELENGTH (nm)

FIG. 1

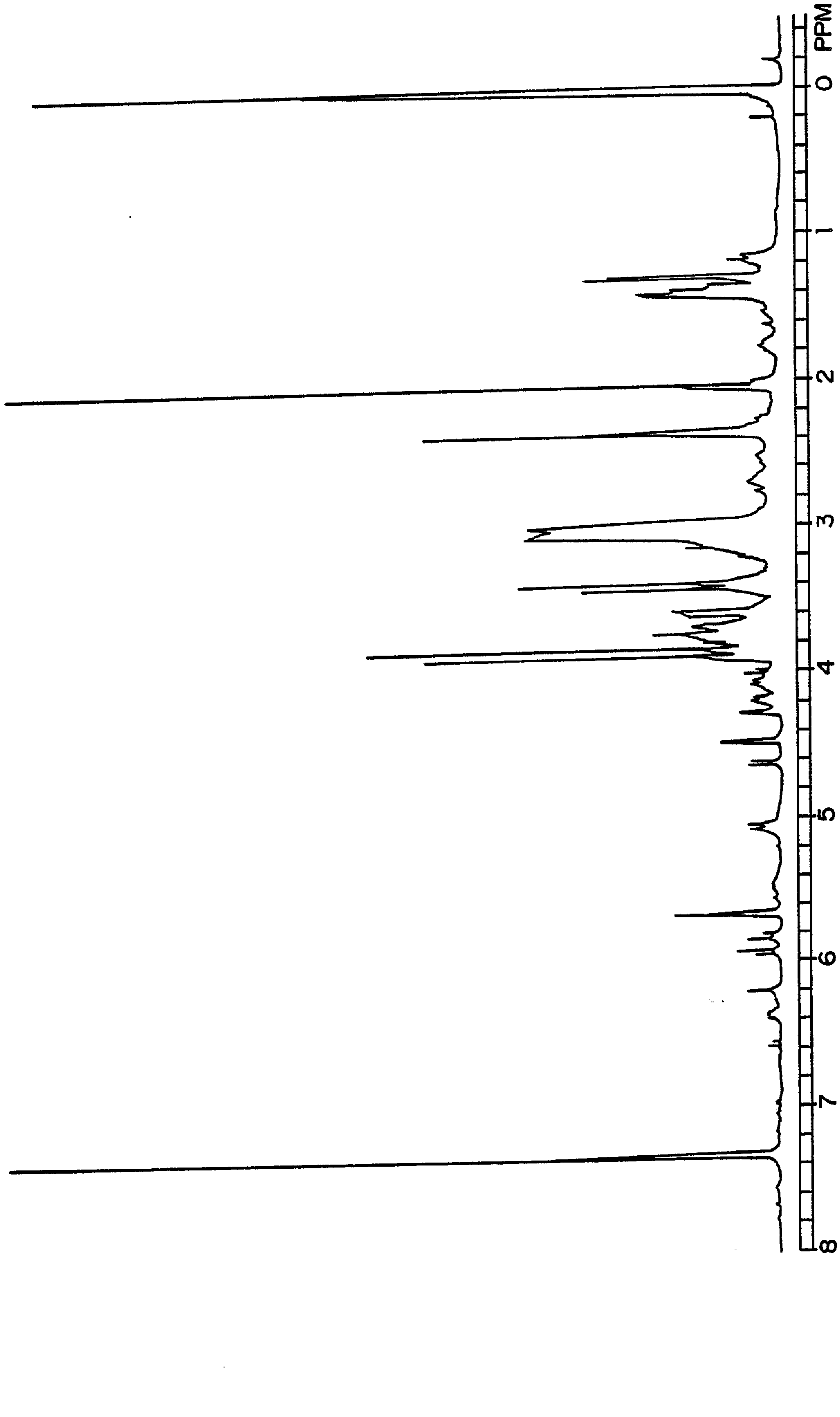


FIG. 2

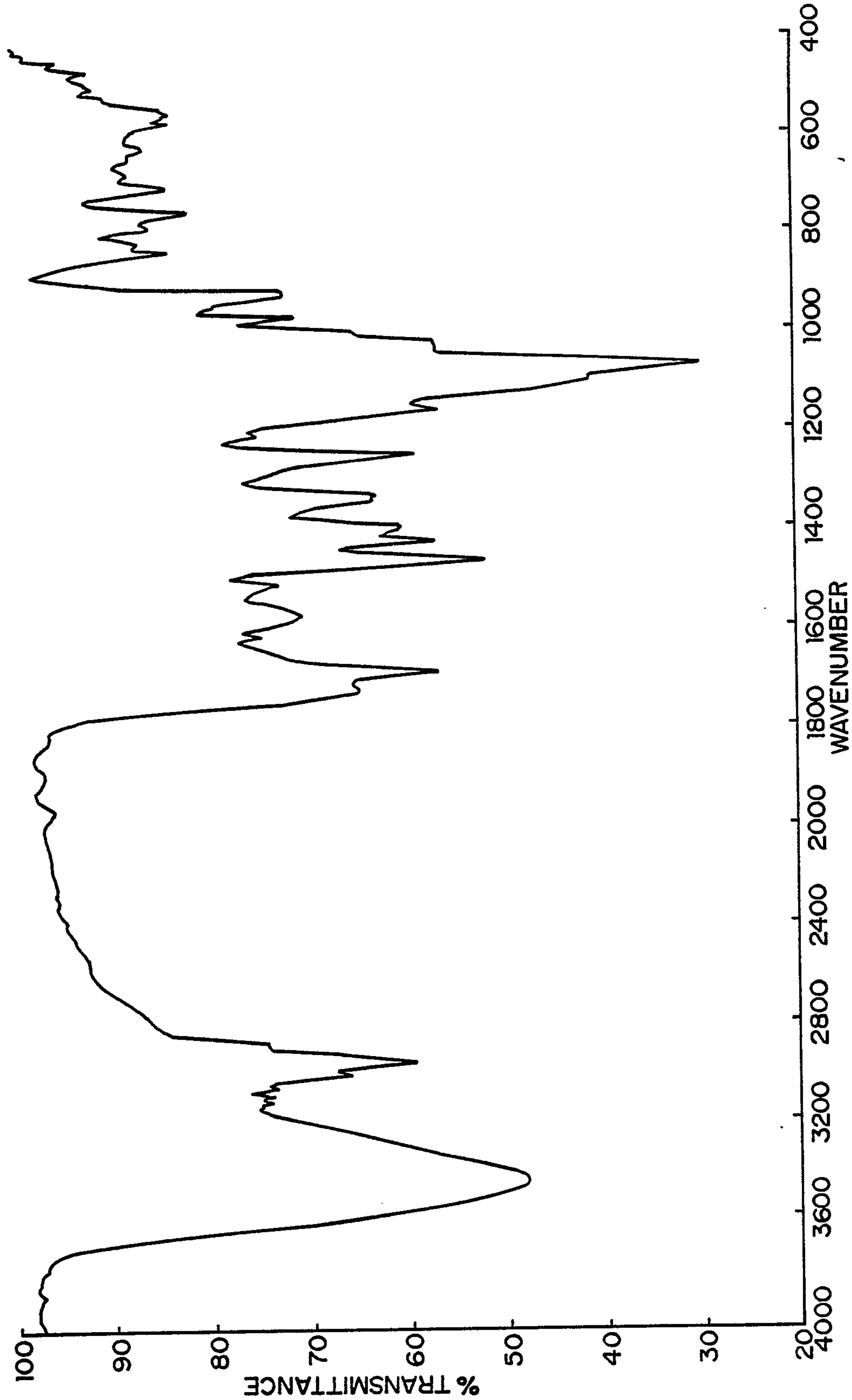
% TRANSMITTANCE
100
90
80
70
60
50
40
30
20
WAVENUMBER
4000
3600
3200
2800
2400
2000
1800
1600
1400
1200
1000
800
600
400

FIG. 3

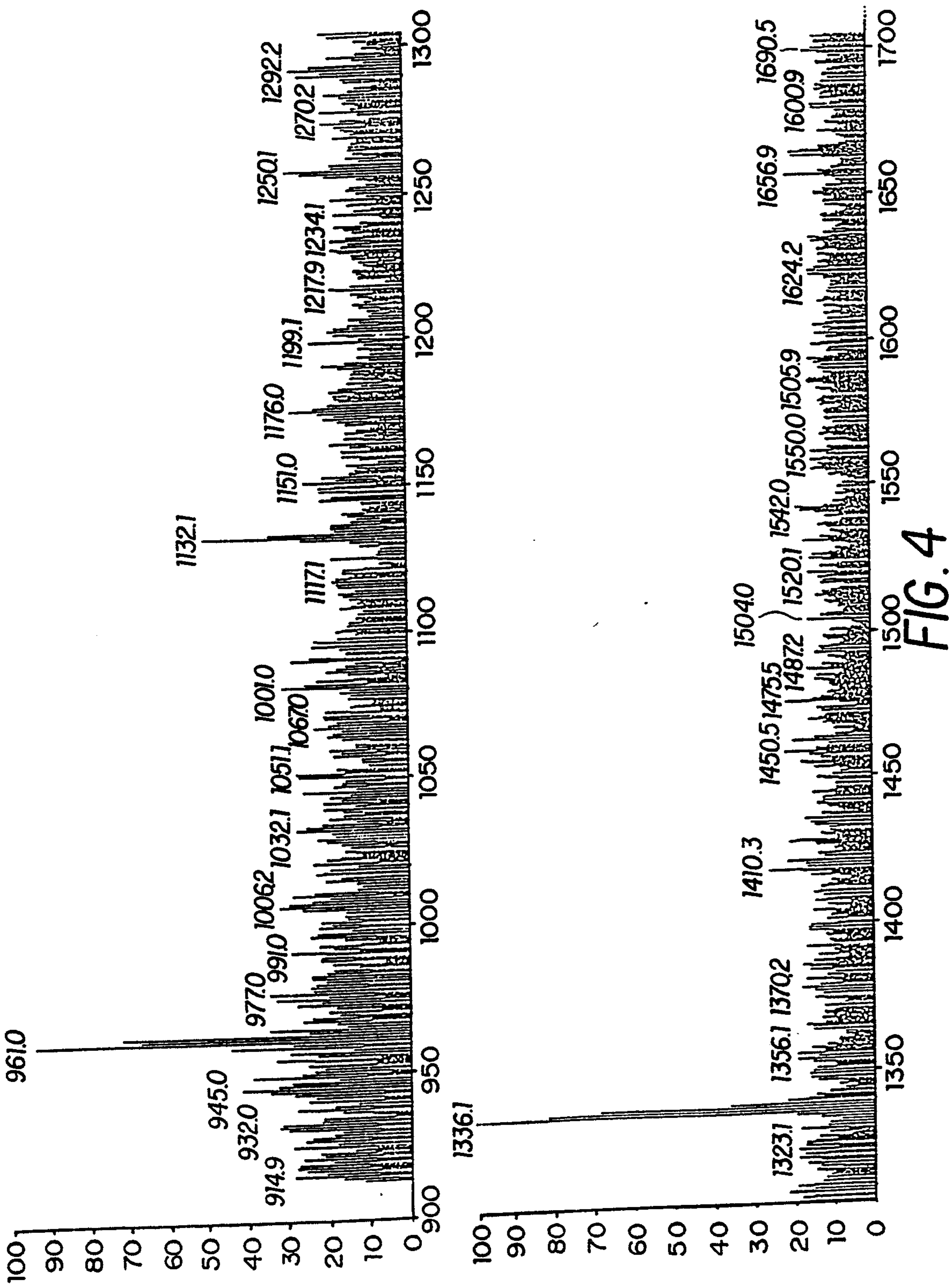
100
90
80
70
60
50
40
30
20
10
0
900
950
1000
1050
1100
1150
1200
1250
1300
914.9
932.0
945.0
961.0
977.0
991.0
1006.2
1032.1
1051.1
1067.0
1001.0
1117.1
1132.1
1151.0
1176.0
1199.1
1217.9
1234.1
1250.1
1270.2
1292.2
1350
1400
1450
1500
1550
1600
1650
1700
1323.1
1336.1
1356.1
1370.2
1410.3
1450.5
1475.5
1487.2
1504.0
1520.1
1542.0
1550.0
1505.9
1624.2
1656.9
1600.9
1690.5
FIG. 4